# EUROPÄISCHE PATENTANMELDUNG

(11) **EP 3 673 888 A1**
(43) Veröffentlichungstag der Anmeldung: **01.07.2020**
(21) Anmeldenummer: 19219158.3
(22) Anmeldetag: 20.12.2019
(51) Int. Cl.: A61J 1/10, A61J 1/20, A61J 1/00, A61M 5/14

(54) **BEUTEL ZUR BEREITSTELLUNG EINES MEDIZINISCHEN WIRKSTOFFS UND SET MIT BEUTEL UND ÜBERLEITSYSTEM**

(30) Priorität: 27.12.2018 DE 102018251757
(71) Anmelder: Eurozyto GmbH, 61462 Königstein (DE)
(72) Erfinder: Rose, Uwe-Bernd, 61462 Königstein (DE)
(74) Vertreter: Patent- und Rechtsanwälte Ullrich & Naumann

(57) **Zusammenfassung**

Ein Beutel dient zur Bereitstellung eines medizinischen Wirkstoffs für eine medizinische Therapie, insbesondere einer Enzyminfusion für eine Enzymersatztherapie oder Antikörperinfusion für eine Antikörpertherapie Der Beutel umfasst einem in mindestens zwei Kammern unterteilten Innenraum, wobei in einer einlassseitigen Wirkstoffkammer der medizinische Wirkstoff, und in einer auslassseitigen Trägerlösungskammer eine für den Wirkstoff geeignete bzw. vom Hersteller vorgeschriebene Trägerlösung, , bereitstellbar ist und wobei zwischen den Kammern eine Flüssigkeitsverbindung derart herstellbar ist, dass bei befüllten Kammern der medizinische Wirkstoff vorzugsweise per Schwerkraft in die Trägerlösungskammer strömt, von der aus die Trägerlösung gemeinsam mit dem medizinischen Wirkstoff über mindestens einen Auslassport per Schwerkraft oder über eine Pumpe auslassbar bzw. förderbar ist.

## Beschreibung

Die Erfindung betrifft einen Beutel zur Bereitstellung eines medizinischen Wirkstoffs für eine medizinische Therapie, insbesondere einer Enzyminfusion für eine Enzymersatztherapie bzw. Antikörperinfusion für eine Antikörpertherapie.

Bei der Erfindung geht es grundsätzlich um die Bereitstellung eines medizinischen Wirkstoffs, ganz gleich mit welcher Indikation. Im Konkreten dient der medizinische Wirkstoff zur medizinischen Therapie, wobei es sich bei dem Wirkstoff um ein Enzym handeln kann, nämlich für eine Enzymersatztherapie bzw. auch um Antikörper für eine Antikörpertherapie.

Im Rahmen einer Enzymersatztherapie werden beim Menschen jene Erkrankungen behandelt, bei denen aufgrund von Gendefekten fehlende Enzyme auf verschiedene Art verabreicht werden. Je nach Art der Erkrankung und in Abhängigkeit vom Zeitpunkt der Diagnose ist der Betroffene dank der Therapie in der Lage, ein annähernd normales Leben zu führen, da der im Organismus fehlende Stoff von außen zugeführt werden kann.

Im Rahmen einer Antikörpertherapie richten sich die Antikörper, die therapeutisch eingesetzt werden, jeweils gegen ganz bestimmte Strukturen im Körper, die bei der Krankheitsentstehung oder im Krankheitsverlauf eine wichtige Rolle spielen. So gibt es spezielle Gruppen von Antikörpern, die bestimmte Rezeptoren, auf denen die Krebszellen andocken, blockieren und so die Zellteilung und damit das Wachstum eines Tumors verringern können. Andere Antikörper lagern sich an Tumorzellen an und lösen dadurch eine Abwehrreaktion aus.

Außerdem gibt es auch Antikörper, die entzündungsfördernde Botenstoffe, die bei Autoimmunerkrankungen wie Rheuma oder Multipler Sklerose eine Rolle spielen, gezielt erkennen und deren Bekämpfung durch das körpereigene Immunsystem einleiten können.

Die Infusion kann kurz vor Verabreichung der entsprechenden Enzyme oder auch Antikörper entweder durch Auflösen einer Trockensubstanz oder durch Zugabe eines fertigen, bereits flüssigen Infusionskonzentrats in eine Trägerlösung gegeben werden.

Werden Einkammerbeutel (Infusionsfertigbeutel mit vorgefüllter Trägerlösung) benutzt, reduziert sich die Haltbarkeit der Infusionslösung in der Regel auf wenige Stunden. Dies ist den entsprechenden Fachinformationen der Präparatehersteller zu entnehmen. Die nur kurze Haltbarkeit ist problematisch und erhöht bei Verfall der Infusionslösung die Kosten ganz erheblich.

Der vorliegenden Erfindung liegt die Aufgabe zugrunde, den hier in Rede stehenden Beutel zur Bereitstellung eines medizinischen Wirkstoffs für eine medizinische Therapie anzugeben, der bei einfachster Konstruktion eine sichere Handhabung gewährleistet, und dies bei maximaler Haltbarkeit der Infusionslösung.

Voranstehende Aufgabe ist durch die Merkmale des Anspruchs 1 gelöst. Danach dient der Beutel zur Bereitstellung eines medizinischen Wirkstoffs für eine medizinische Therapie und ist insbesondere zur Enzyminfusion für eine Enzymersatztherapie oder Antikörperinfusion für eine Antikörpertherapie geeignet. Der Innenraum des Beutels ist in mindestens zwei Kammern unterteilt, wobei in einer einlassseitigen Wirkstoffkammer der medizinische Wirkstoff, und in einer auslassseitigen Trägerlösungskammer eine für den Wirkstoff geeignete oder vom Hersteller vorgeschriebene Trägerlösung, bereitstellbar ist. Zwischen den Kammern ist eine Flüssigkeitsverbindung derart herstellbar, dass bei befüllten Kammern der medizinische Wirkstoff vorzugsweise per Schwerkraft in die Trägerlösungskammer strömt, von der aus die Trägerlösung gemeinsam mit dem medizinischen Wirkstoff über mindestens einen Auslassport per Schwerkraft oder über eine Pumpe auslassbar bzw. förderbar ist.

Erfindungsgemäß wird erreicht, dass bei einfacher Handhabung eine Verabreichung eines zu infundierenden Enzyms/Antikörpers möglich ist, wobei der Beutel beispielsweise in einem Reinraum befüllt werden kann. Der Beutel ist im befüllten Zustand gebrauchsfertig. Sein Inhalt muss im Nachgang zu der Befüllung nicht mehr manipuliert werden.

Erfindungsgemäß ist ein System realisiert, wodurch es möglich ist, eine Wirklösung von der Trägerlösung vollständig zu trennen und erst kurz vor der Anwendung zu aktivieren, wenngleich beides - Wirklösung und Trägerlösung - bereits in den Beutel gefüllt sind. Dadurch ist es möglich, die Haltbarkeitsdaten der Hersteller, wie sie in den bisherigen Fachinformationen angegeben sind oder unter der Voraussetzung, dass die jeweiligen Stabilitäten der Enzyminfusions-/Antikörperinfusionslösungen vorher geprüft werden, zu verlängern.

An diese Stelle sei angemerkt, dass ein Beutelmaterial zu verwenden ist, wonach der in der Wirkstoffkammer befindliche Wirkstoff, beispielsweise das dort befindliche Enzym/Antikörper, bis zur Zusammenführung mit der Trägerlösung keinen Aktivitätsverlust aufweist.

Durch die konstruktiven Gegebenheiten des erfindungsgemäßen Beutels mit mindestens zwei Kammern - Wirkstoffkammer und Trägerlösungskammer - lässt sich eine Flüssigkeitsverbindung herstellen, wonach die beiden Flüssigkeiten - Wirkstoff bzw. Wirkstofflösung und Trägerlösung getrennt gelagert und bedarfsgerecht ineinanderlaufen können, bzw. bedarfsgerecht vorgefüllt werden können.

Parallel zu der Wirkstoffkammer oder dieser vorgeschaltet kann eine weitere Kammer vorgesehen sein, nämlich eine Spülkammer zur Bereitstellung einer Spüllösung. Die Spüllösung dient zum Spülen der Wirkstoffkammer, wobei dazu eine Strömungsverbindung zwischen der Spülkammer und der Wirkstoffkammer hergestellt wird, derart, dass die Spüllösung vorzugsweise per Schwerkraft in die Wirkstoffkammer und von dort mit Resten des Wirkstoffs in die Trägerlösungskammer gelangt.

Die Bereitstellung einer Spüllösung in einer separaten Spülkammer ist von ganz besonderem Vorteil, da es sich bei den Wirkstoffen, beispielsweise bei einer Enzymlösung alternativ Antikörperlösung, um teilweise minimal dosiertes Produkt handeln kann, welches und aufgrund eventuell eines hohen Preises vollständig dem Patienten zugeführt werden muss. Umso mehr macht es Sinn, dass man die in der Wirkstoffkammer verbleibenden Reste des Wirkstoffs zur Verabreichung herausspült, nämlich mittels der Spüllösung, bei der es sich um eine ähnliche oder identische Flüssigkeit wie bei der Trägerlösung handeln kann.

Im ungebrauchten Zustand besteht zwischen den Kammern des Beutels keine Strömungsverbindung, sind die Kammern vielmehr voneinander getrennt. Zur Herstellung der Flüssigkeitsverbindung zwischen den Kammern ist ein öffenbarer Verbindungskanal vorgesehen, wobei es sich dabei um ein Trennventil handeln kann. Im Konkreten kann es sich dabei um ein aus Glas und/oder Kunststoff bestehendes Bruchventil handeln, welches von außerhalb des Beutels geöffnet bzw. gebrochen werden kann, wodurch eine Strömungsverbindung zwischen den jeweiligen Kammern hergestellt wird.

An dieser Stelle sei angemerkt, dass auch mehr als eine Wirkstoffkammer vorgesehen sein kann, nämlich dann, wenn es darum geht, unterschiedliche Wirkstoffe entweder miteinander bzw. untereinander zu verbinden und/oder diese der Trägerlösung zuzuführen. Ungeachtet dessen und der Einfachheit halber ist nachfolgend von lediglich einer Wirkstoffkammer die Rede.

Geht man von einem befüllten Beutel der erfindungsgemäßen Art aus, so befindet sich in der Wirkstoffkammer die Wirkstofflösung, beispielsweise eine Enzymlösung zur Enzyminfusion oder eine Antikörperlösung zur Antikörpertherapie. In der Trägerlösungskammer befindet sich eine geeignete Trägerlösung. Zum Lagern sind die beiden Flüssigkeiten voneinander getrennt. Maximale Haltbarkeit ist gewährleistet.

Vor bzw. zur Verabreichung oder Anwendung wird der Verbindungskanal zwischen der Wirkstoffkammer und der Trägerlösungskammer geöffnet, so dass der Wirkstoff in die Trägerlösung fließen bzw. strömen kann. Dadurch findet eine Verdünnung des Wirkstoffs statt.

Die Mischung aus Wirkstoff und Trägerlösung steht dann zur Infusion bereit.

Wie bereits zuvor ausgeführt, kann die Befüllung des Beutels unter Reinraumbedingungen erfolgen. Zum Befüllen der Kammern kann jeweils ein Einlass zum vorzugsweise individuellen Befüllen der jeweiligen Kammer vorgesehen sein, wobei der Einlass als vorzugsweise absperrbarer bzw. schließbarer Schlaucheinlass, Luer-Lock-Port ausgeführt sein kann.

Auslassseitig können zwei oder mehrere absperrbare Auslassports mit unterschiedlichen Anschlussmöglichkeiten vorgesehen sein, beispielsweise für einen Luer-Anschluss, einen Schraubanschluss, einen Steckanschluss, einen Spike Port, etc. Die Auslassports sind vorzugsweise mit der Trägerlösungskammer unmittelbar oder mittelbar strömungsverbunden. Vor dem jeweiligen Port kann eine die Strömung beeinflussende Schnecke oder dergleichen zur Begünstigung einer weiterreichenden Vermischung der Flüssigkeiten vorgesehen sein.

Die zuvor erörterten Kammern können derart angeordnet und ausgebildet sein, dass nach Aufhängen des Beutels auf/an der Einlassseite und nach Herstellung einer Flüssigkeitsverbindung zunächst zwischen der Wirkstoffkammer und der Trägerlösungskammer der medizinische Wirkstoff, beispielsweise die Enzymlösung/ Antikörperlösung, per Schwerkraft in die Trägerlösungskammer gelangt. Von dort gelangt die Mischung von Wirkstoff und Trägerlösung zum Auslassport und kann aus dem Beutel ausströmen. Durch Herstellen einer Flüssigkeitsverbindung zwischen der Spülkammer und der Wirkstoffkammer gelangt die Spüllösung in die Wirkstoffkammer und gelangt von dort gemeinsam mit Resten des Wirkstoffs in die Trägerlösungskammer. Von dort aus strömt der "Rest" ebenfalls durch den geöffneten Port, wodurch der Wirkstoff optimal ausbringbar ist.

Zum Ausbringen der Enzymlösung/Antikörperlösung ist es von Vorteil, wenn der Beutel - mit der Wirkstoffkammer oben und der Trägerlösungskammer unten - aufhängbar ist. Dazu ist mindestens ein Aufhängepunkt zum Befestigen bzw. Aufhängen des Beutels vorgesehen, insbesondere in Form eines Durchgangs durch einen abseits des Innenraums ausgebildeten Randbereich des Beutels. Dieser Durchgang könnte im Bereich einer Materialverstärkung des Beutels bzw. Beutelrandes ausgebildet sein, um ein sicheres Aufhängen eines im gefüllten Zustand schweren Beutels gefahrlos zu ermöglichen.

Bereits zuvor ist ausgeführt worden, dass der Wirkstoff gerade im Rahmen einer Enzymersatztherapie/Antikörpertherapie teuer sein kann. Daher sollte die Wirkstoffkammer derart geformt und ausgebildet sein, dass eine vollständige oder annähernd vollständige Entleerung der Kammer begünstigt bzw. möglich ist. Vorzugsweise im Randbereich der unteren Begrenzung der Kammer könnten fallende Flanken, Schrägen, etc. in Richtung des jeweiligen Verbindungskanals oder in Richtung des Auslassports ausgebildet sein, so dass per Schwerkraft eine nahezu völlige Entleerung der Kammer möglich ist.

Auch ist es denkbar, dass neben der Form der Kammer das Material und/oder die Oberflächenbeschaffenheit zumindest der Wirkstoffkammer derart ausgebildet ausgelegt ist/sind, so dass eine annähernd vollständige Entleerung der Kammer begünstigt ist. Bei der Materialwahl und/oder Oberflächenbeschaffenheit ist darauf zu achten, dass die Flüssigkeit mehr oder weniger von der inneren Oberfläche der Kammer "perlt", nämlich aufgrund der jeweiligen Oberflächen- und Grenzflächenenergien und einer daraus resultierenden schlechten Benetzung.

Wenngleich der Inhalt der Kammern, insbesondere der Inhalt der Trägerlösungskammer, durch Schwerkraft entleerbar ist, kann eine vorzugsweise elektrische, insbesondere mobile Pumpe vorgesehen sein, die zum Fördern der Mischung aus Wirkstoff und Trägerlösung dient. Dadurch wird beispielsweise die Verabreichung einer Enzyminfusion/ Antikörperinfusion vereinfacht oder erleichtert.

Der Beutel kann aus einem polymeren Material bestehen, beispielsweise aus einer flexiblen polymeren Folie. Der Innenraum des Beutels wird durch Falten oder Umschlagen der Folie und randseitiges Verschweißen und/oder durch randseitiges Verkleben und/oder durch randseitige Klemmmittel gebildet. Gleiches gilt für die Trennung zwischen den Kammern, die gemeinsam den Innenraum des Beutels definieren. Die zwischen den Kammern zur Strömungsverbindung dienenden Kanäle können in die jeweilige Naht bzw. Trennung zwischen den Kammern eingebracht/eingebunden werden, vorzugsweise bereits während der Fertigung des Beutels.

Ebenso ist es denkbar, dass die Folie als endseitig verschweißter oder verklebter Schlauch, vorzugsweise aus einem Polymerschlauch, gebildet ist. Die Trennung zwischen den Kammern wird gemäß den voranstehenden Ausführungen hergestellt.

Entsprechend dem nebengeordneten Anspruch 14 ist der Beutel im befüllten Zustand beansprucht, nämlich mit in den Kammern befindlichem Wirkstoff (in der Wirkstoffkammer) und Trägerlösung (in der Trägerlösungskammer). Gleiches gilt für die Spüllösung in der Spülkammer. Die konkrete Konstruktion des Beutels ist mit den abhängigen Ansprüchen 2 bis 13 beansprucht und voranstehend beschrieben.

Der weiter nebengeordnete Anspruch 15 beansprucht ein Set zur Bereitstellung eines medizinischen Wirkstoffs für eine medizinische Therapie, insbesondere einer Enzyminfusion für eine Enzymtherapie oder Antikörperinfusion für eine Antikörpertherapie Der Beutel, mit und ohne Flüssigkeiten, wird gemeinsam mit einem ankoppelbaren Überleitsystem zur Verabreichung des medizinischen Wirkstoffs beansprucht, gemeinsam oder jeweils einzeln in einer geschlossenen Verpackung.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel eines erfindungsgemäßen Beutels mit drei Kammern,
- Fig. 2: in einer schematischen Ansicht den Beutel aus Figur 1 mit angeschlossenem Überleitsystem
- Fig. 3: in einer schematischen Ansicht ein weiteres Ausführungsbeispiel eines erfindungsgemäßen Beutels.

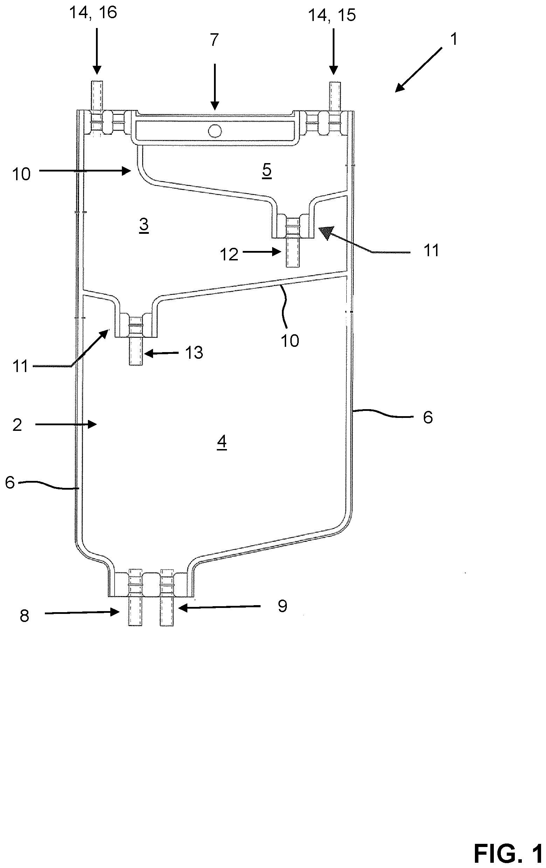

Figur 1 zeigt ein Ausführungsbeispiel eines erfindungsgemäßen Beutels 1 zur Bereitstellung einer Enzyminfusion oder Antikörperinfusion. Bei dem Beutel 1 handelt es sich um einen polymeren Mehrkammerbeutel, im Konkreten mit einem Innenraum 2, der drei Kammern umfasst, nämlich eine Wirkstoffkammer 3 mit einer Enzymlösung oder Antikörperlösung, einer Trägerlösungskammer 4 mit einer Trägerlösung und einer Spülkammer 5 mit einer Spüllösung. Bei der Spüllösung kann es sich um die gleiche Flüssigkeit wie bei der Trägerlösung handeln.

Die drei Kammern 3, 4 und 5 sind zunächst voneinander getrennt.

Der Beutel 1 ist aus einer umgeschlagenen flexiblen Kunststofffolie gebildet, mit einer den Innenraum 2 begrenzenden Schweißnaht 6.

Im oberen Bereich ist die Folie umgeschlagen und dadurch nahtlos geschlossen. Andere Abgrenzungen und/oder Verschlussmechanismen sind denkbar.

Zur Befestigung bzw. zum Aufhängen des Beutels 1 ist ein Durchgang 7 als Befestigungspunkt vorgesehen, wobei der Beutel 1 mit mehreren Durchgänge 7 ausgestattet sein kann. Durch diese Maßnahme ist eine Flüssigkeitsentnahme aus dem Beutel 1 bzw. den Kammern 3, 4 und 5 per Schwerkraft möglich, wobei der Beutel 1 derart aufzuhängen ist, dass die Entnahmestelle bzw. die zur Entnahme dienenden Ports 8, 9 zum Erdboden bzw. nach unten weisen.

Die Kammern 3, 4 und 5 sind durch Kammerwandungen 10 voneinander getrennt, wobei zwei Kammerwandungen 10 zur Bildung von insgesamt drei Kammern 3, 4 und 5 notwendig sind. In die jeweilige Kammerwandung 10 ist ein Kanal 11 integriert, der zur Flüssigkeitsverbindung zwischen den Kammern 3, 4 und 5 dient.

Zur Vorbereitung der Infusion wird eine Flüssigkeitsverbindung zwischen der Wirkstoffkammer 3 und der Trägerlösungskammer 4 hergestellt, indem nämlich der in die Kammerwandung 10 integrierte Kanal 11 geöffnet wird. Die Wirkstofflösung läuft nach Öffnen des Kanals 11 in die Trägerlösungskammer 4, wo eine Vermischung von Wirkstoff und Trägerlösung stattfindet.

Anschließend wird die Kammerwand 10 zwischen der Spülkammer 5 und der Wirkstoffkammer 3 geöffnet, wodurch Reste des Wirkstoffs aus der Wirkstoffkammer 3 heraus in die Trägerlösungskammer 4 gespült werden.

Zum Öffnen des jeweiligen Kanals 11 werden die den Kanal definierenden Röhrchen 12, 13 geöffnet bzw. zerstört, so dass die zuvor erörterte Flüssigkeitsverbindung hergestellt wird.

Die Röhrchen 12, 13 sind so gestaltet, dass durch Abknicken des Röhrchens 12 ,13 der Kanal 11 freigegeben ist und die jeweiligen Flüssigkeiten in der jeweils darunter angeordneten Kammer ineinanderlaufen. Sind die Röhrchen 12, 13 geknickt bzw. zerstört, lässt sich der so geschaffene Durchgang bzw. die dadurch hergestellte Strömungsverbindung nicht wieder schließen.

Das zur Strömungsverbindung gebrochene Röhrchen 12, 13 ist gleichzeitig ein Indikator für die erste Ingebrauchnahme bzw. die Strömungsverbindung zwischen zwei Kammern und ist daher im Sinne eines "Frischesiegels" zu verstehen.

Den Kammern 3, 4 und 5 ist jeweils ein Anschlussstück 14 zum Befüllen oder entleeren des Kammerinhalts zugeordnet. Das Anschlussstück 14 kann als Luer-Lock Anschluss 15 oder 16 ausgebildet sein. Damit ist nicht nur eine ordnungsgemäße Befüllung oder Applikation der jeweiligen Flüssigkeit möglich, sondern auch ein Auffüllen oder Nachfüllen einer benötigten Flüssigkeit, falls dies medizinisch notwendig ist.

Die unterste Kammer, d.h. die Trägerlösungskammer 4, kann zwei unterschiedliche Ports 8, 9 aufweisen, beispielsweise einen Luer-Lock Anschluss und einem weiteren Luer-Lock Anschluss oder einen Spike-Port, diese Ports ermöglichen zusätzlich den Anschluss einer Infusionspumpe. Durch diese sehr unterschiedlichen Vorkehrungen ist eine flexible Anschlussmöglichkeit des Beutels 1 zur Bereitstellung der Enzyminfusion oder einer Antikörperinfusion gegeben. Auch ist eine redundante Anschlussmöglichkeit realisiert, wenn am Einsatzort das komplementäre Gegenstück zu den am Beutel 1 ausgebildeten Anschlüsse defekt oder gar nicht erst vorhanden sein sollte. Eine maximale Variabilität/Flexibilität ist gegeben.

Der Beutel 1 kann unterschiedliche Volumina aufweisen. Beispielhaft kann die obere Spülkammer 50 ml Spüllösung beinhalten. Als Zugang ist ein Luer-Lock Anschluss 15 vorgesehen.

Die darunter angeordnete Wirkstoffkammer 3 (Enzym/Antikörperkammer) kann beispielsweise 100 ml aufweisen. Auch hier kann ein Luer-Lock Anschluss 16 vorgesehen sein.

Der Beutel 1 kann beispielsweise eine Länge von 300 mm bis 400 mm, vorzugsweise eine Länge von 350 mm haben. Die Breite des Beutels 1 kann bei 150 mm bis 200 mm, vorzugsweise bei 170 mm, liegen.

Die drei Kammern - Wirkstoffkammer 3, Trägerlösungskammer 4 und Spülkammer 5 -, haben unterschiedliche Größe bzw. Volumina und sind bei dem hier gewählten Ausführungsbeispiel gestuft dimensioniert. So lassen sich in dem Beutel 1 unterschiedliche Flüssigkeiten mit den konkret benötigten Mengen speichern/lagern. Außerdem weist die Wirkstoffkammer 3 ein größeres Volumen als die Spülkammer 5 auf, wobei die Spülkammer 5 zur Spülung des Beutels 1 bzw. des Innenraums 2 kurz vor oder nach Infusionsende dient.

Die größte Kammer, nämlich die Trägerlösungskammer 4, kann ein Fassungsvermögen von 300 mL bis 600 mL aufweisen, vorzugsweise im Bereich von bis zu 500 ml, je nach Bedarf.

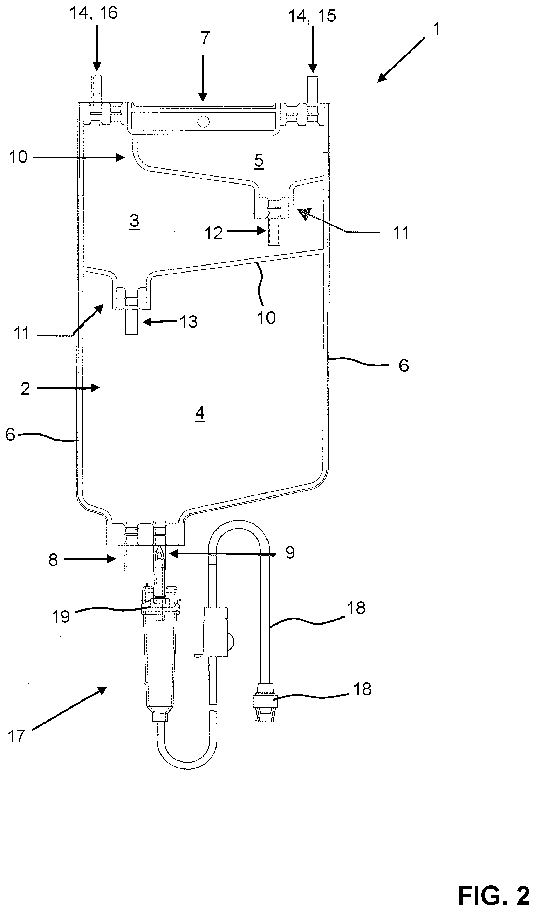

Figur 2 zeigt den Beutel aus Figur 1, ergänzt zu einem Set zur Bereitstellung einer Enzyminfusion/Antikörperinfusion. Das Set umfasst ein erfindungsgemäßen Beutel 1 mit den zuvor erörterten Flüssigkeiten in den drei Kammern 3, 4 und 5. Das Set umfasst zudem ein nach den Vorgaben des behandelnden Arztes gestaltest Infusionssystem 17 mit den jeweiligen Anschlüssen 19 und ggf. ein Pumpensystem.

Das Infusionssystem 17 dient als Überleitsystem, das über eine Schlauchleitung 18 und entsprechende Anschlüsse 19 verfügt. Das System kann entweder als Schwerkraftsystem oder als Spezialsystem zum Anschluss an eine Infusionspumpe ausgelegt sein. An einem Ende des Infusionssystems 17 ist ein Anschlussstück zum Anschluss an dem Beutel 1 und an dem anderen Ende ist ein Anschlussstück für eine intravenöse Applikation vorgesehen.

Der Beutel 1 und das Infusionssystem 17 werden in einer geschlossenen und sterilen Verpackung bereitgestellt, die in den Figuren nicht gezeigt ist. Die Verpackung kann als Beutel aus übereinanderliegenden Kunststofffolien gefertigt sein, vorzugsweise mit umlaufender Schweißnaht. Zumindest ein Teil der Schweißnaht kann mit einer Art Sollbruchstelle zum Aufreißen ausgebildet sein, nämlich im Sinne einer sogenannten Peelnaht. Daher ist es möglich, eine üblicherweise sterile Verpackung auf einfache Art und Weise zu öffnen, um die Bestandteile des Sets - Beutel 1 und Infusionssystem 17 bzw. Überleitsystem - zu entnehmen. Da der Inhalt des Sets - Beutel 1 und Überleitsystem 17 - im sterilen und anwendungsfertigen Zustand bereitstellt wird, ist eine unmittelbare und zügige Verabreichung der Enzyminfusion/Antikörperinfusion möglich, ohne weiteren technischen Aufwand. Die Verpackung muss lediglich geöffnet werden. Nach Entnahme der Bestandteile des Sets lässt sich die Infusion applizieren.

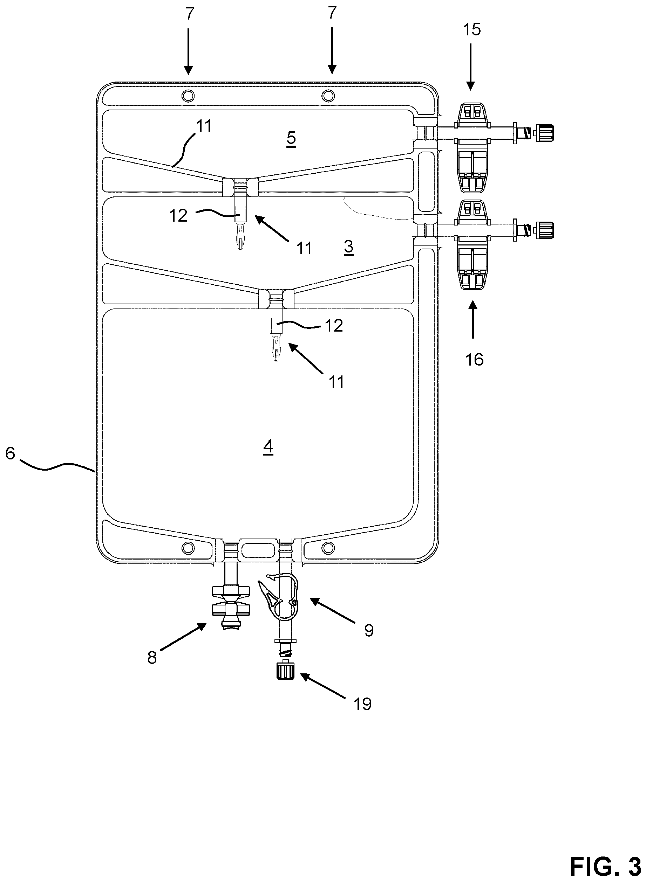

Figur 3 zeigt ein weiteres Ausführungsbeispiel eines erfindungsgemäßen Beutels 1, wobei dort gegenüber dem Ausführungsbeispiel aus den Figuren 1 und 2 der Innenraum 2 anders gestaltet bzw. aufgeteilt ist. Im Konkreten sind dort die Kammern - Wirkstoffkammer 3, Trägerlösungskammer 4 und Spülkammer 5 - übereinander angeordnet, wobei umlaufende bzw. seitliche Randbereich, die verstärkt sein können, zur Stabilisierung des Beutels 1 dienen. Zur Vermeidung von Wiederholungen sei ansonsten auf die Ausführungen zu den Figuren 1 und 2 verwiesen.

Hinsichtlich weiterer vorteilhafter Ausgestaltungen der erfindungsgemäßen Lehre wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Lehre lediglich zur Erörterung der beanspruchten Lehre dienen, diese jedoch nicht auf die Ausführungsbeispiele einschränken.

### Bezugszeichenliste

- 1: Beutel
- 2: Innenraum
- 3: Wirkstoffkammer
- 4: Trägerlösungskammer
- 5: Spülkammer
- 6: Schweißnaht
- 7: Durchgang
- 8: Port
- 9: Port
- 10: Kammerwandung
- 11: Kanal
- 12: Röhrchen
- 13: Röhrchen
- 14: Anschlussstück
- 15: Luer-Lock-Anschluss
- 16: Luer-Lock-Anschluss
- 17: Infusionssystem, Überleitsystem
- 18: Schlauchleitung
- 19: Anschlüsse

## Patentansprüche

1. Beutel zur Bereitstellung eines medizinischen Wirkstoffs für eine medizinische Therapie, insbesondere einer Enzyminfusion für eine Enzymersatztherapie/Antikörperinfusion für eine Antikörpertherapie, mit einem in mindestens zwei Kammern unterteilten Innenraum, wobei in einer einlassseitigen Wirkstoffkammer der medizinische Wirkstoff, und in einer auslassseitigen Trägerlösungskammer eine Trägerlösung, insbesondere eine Glukose- und/oder Kochsalzlösung, bereitstellbar ist und wobei zwischen den Kammern eine Flüssigkeitsverbindung derart herstellbar ist, dass bei befüllten Kammern der medizinische Wirkstoff vorzugsweise per Schwerkraft in die Trägerlösungskammer strömt, von der aus die Trägerlösung gemeinsam mit dem medizinischen Wirkstoff über mindestens einen Auslassport per Schwerkraft oder über eine Pumpe auslassbar bzw. förderbar ist.

2. Beutel nach Anspruch 1, **dadurch gekennzeichnet, dass** parallel zu der Wirkstoffkammer oder dieser vorgeschaltet eine Spülkammer zur Bereitstellung einer Spüllösung vorgesehen ist, wobei zum Spülen der Wirkstoffkammer zwischen der Spülkammer und der Wirkstoffkammer eine Flüssigkeitsverbindung herstellbar ist, so dass die Spüllösung vorzugsweise per Schwerkraft in die Wirkstoffkammer und von dort mit Resten des Wirkstoffs in die Trägerlösungskammer gelangt.

3. Beutel nach Anspruch 2, **dadurch gekennzeichnet, dass** es sich bei der Spüllösung um die gleiche Flüssigkeit wie bei der Trägerlösung handelt.

4. Beutel nach einem der Ansprüche 1 bis 3, **dadurch gekennzeichnet, dass** zur Flüssigkeitsverbindung zwischen den Kammern jeweils mindestens ein öffenbarer Verbindungskanal, vorzugsweise mit einem Trennventil, insbesondere mit einem aus Glas und/oder Kunststoff bestehenden Bruchventil, vorgesehen ist.

5. Beutel nach einem der Ansprüche 1 bis 4, **dadurch gekennzeichnet, dass** zumindest einer der Kammern, vorzugsweise zwei der Kammern oder allen Kammern, mindestens ein Einlass zum vorzugsweise individuellen Befüllen und/oder Entleeren der jeweiligen Kammer zugeordnet ist, wobei der Einlass als vorzugsweise absperrbarer bzw. schließbarer Schlaucheinlass (5), Luer-Lock-Port ausgeführt sein kann.

6. Beutel nach einem der Ansprüche 1 bis 5, **dadurch gekennzeichnet, dass** zwei oder mehrere Auslassports mit unterschiedlichen Anschlussmöglichkeiten vorgesehen sind, beispielsweise für einen Luer-Anschluss, einen Schraubanschluss, einen Steckanschluss oder einen Spike-Anschluss, wobei die Auslassports vorzugsweise mit der Trägerlösungskammer unmittelbar oder mittelbar strömungsverbunden sind.

7. Beutel nach einem der Ansprüche 1 bis 6, **dadurch gekennzeichnet, dass** die Kammern derart angeordnet und ausgebildet sind, dass nach Aufhängen des Beutels auf/an der Einlassseite und nach Herstellung einer Flüssigkeitsverbindung zwischen der Wirkstoffkammer und der Trägerlösungskammer der medizinische Wirkstoff per Schwerkraft in die Trägerlösungskammer und von dort durch den geöffneten Auslassport strömt und dass durch Herstellen einer Flüssigkeitsverbindung zwischen der Spülkammer und der Wirkstoffkammer die Spüllösung durch die Wirkstoffkammer und gemeinsam mit Resten des Wirkstoffs in die Trägerlösungskammer und von dort ebenfalls durch den geöffneten Port gelangt.

8. Beutel nach einem der Ansprüche 1 bis 7, **dadurch gekennzeichnet, dass** mindestens ein Aufhängepunkt zum Befestigen des Beutels, insbesondere in Form eines Durchgangs durch einen Randbereich des Beutels, vorgesehen ist, vorzugsweise im Bereich einer Materialverstärkung des Beutels bzw. Beutelrandes.

9. Beutel nach einem der Ansprüche 1 bis 8, **dadurch gekennzeichnet, dass** zumindest die Wirkstoffkammer derart geformt und ausgebildet ist, dass eine vollständige oder annähernd vollständige Entleerung der Kammer begünstigt ist, wobei vorzugsweise im Bereich der unteren Begrenzung der jeweiligen Kammer fallende Flanken, Schrägen, etc. in Richtung des jeweiligen Verbindungskanals oder in Richtung des Auslassports ausgebildet sind.

10. Beutel nach einem der Ansprüche 1 bis 9, **dadurch gekennzeichnet, dass** die Form und/oder das Material und/oder die Oberflächenbeschaffenheit zumindest der Wirkstoffkammer derart ausgebildet ist/sind, dass eine annähernd vollständige Entleerung der Kammer begünstigt ist.

11. Beutel nach einem der Ansprüche 1 bis 10, **dadurch gekennzeichnet, dass** der Inhalt der Kammern, insbesondere der Inhalt der Trägerlösungskammer, durch Schwerkraft und/oder durch eine vorzugsweise elektrische, insbesondere mobile Pumpe förderbar und somit verabreichbar ist.

12. Beutel nach einem der Ansprüche 1 bis 11, **dadurch gekennzeichnet, dass** der Beutel aus einer flexiblen, vorzugsweise polymeren Folie hergestellt ist und die Kammern durch Falten oder Umschlagen der Folie und randseitige Schweißnähte und/oder durch randseitiges Kleben und/oder durch randseitige Klemmmittel gebildet sind.

13. Beutel nach einem der Ansprüche 1 bis 12, **dadurch gekennzeichnet, dass** die Folie als endseitig verschweißter oder verklebter Schlauch, vorzugsweise aus einem Polymerschlauch, gebildet ist.

14. Beutel zur Bereitstellung eines medizinischen Wirkstoffs für eine medizinische Therapie, insbesondere einer Enzyminfusion für eine Enzymersatztherapie oder Antikörperinfusion für eine Antikörpertherapie, mit einem in mindestens zwei Kammern unterteilten Innenraum, wobei in einer einlassseitigen Wirkstoffkammer der medizinische Wirkstoff, und in einer auslassseitigen Trägerlösungskammer eine Trägerlösung, insbesondere eine Glukose- und/oder Kochsalzlösung, enthalten sind und wobei zwischen den Kammern eine Flüssigkeitsverbindung derart herstellbar ist, dass der medizinische Wirkstoff vorzugsweise per Schwerkraft in die Trägerlösungskammer strömt, von der aus die Trägerlösung gemeinsam mit dem medizinischen Wirkstoff über mindestens einen Auslassport per Schwerkraft oder über eine Pumpe auslassbar ist, wobei der Beutel nach einem der Ansprüche 2 bis 13 ausgebildet sein kann.

15. Set zur Bereitstellung eines medizinischen Wirkstoffs für eine medizinische Therapie, insbesondere einer Enzyminfusion für eine Enzymersatztherapie oder Antikörperinfusion für eine Antikörpertherapie, umfassend einen Beutel nach einem der Ansprüche 1 bis 14 und ein mit dem Beutel koppelbares Überleitsystem zur Verabreichung des medizinischen Wirkstoffes, wobei der Beutel und das Überleitsystem gemeinsam oder jeweils einzeln in einer geschlossenen Verpackung aufgenommen sind.
